# EUROPEAN PATENT APPLICATION

(11) **EP 1 300 137 A2**
(43) Date of publication of application: **09.04.2003**
(21) Application number: 02021180.1
(22) Date of filing: 24.09.2002
(51) Int. Cl.: A61K 7/42

(54) **Combinations of sunscreens**

(30) Priority: 02.10.2001 IT MI20012037
(71) Applicant: 3V SIGMA S.p.A, 20121 Milano (IT)
(72) Inventor: Quintini, Massimo, 20121 Milano (IT)
(74) Representative: Minoja, Fabrizio, Dr.

(57) **Abstract**

Combinations of sunscreens comprising one or more anti UV-B filters and one or more anti UV-A filters and compositions containing them.

## Description

The present invention relates to combinations of sunscreens comprising one or more anti UV-B filters and one or more anti UV-A filters as hereinafter defined and to compositions containing them.

It is well known that sun radiations ranging from 280 to 400 nm are noxious for human skin; in particular those with a wavelength from 280 to 320 nm, the socalled UV-B radiation, cause erythema and cutaneous sunburns, whose severity depends on the kind of the skin and on the duration of the exposition to sunlight. It has been ascertained that also radiations ranging from 320 to 400 nm, the so called UV-A radiations, which were considered innocuous and only responsible of skin tanning, are, as a matter of fact, harmful, in that they can cause damages to elastine and collagene, with consequent ageing of the skin as well as a number of phototoxic and photoallergic reactions. It is also known that UV-radiations can cause damages to DNA.

A great number of combinations of anti UV-A and anti UV-B sunscreens are disclosed in the literature and in patents. For example, GB 2 198 944, WO 91/11989 and WO 94/04131 disclose combinations of benzylidenecamphor derivatives or cyanoacrylates (anti UV-B filters) and dibenzoylmethane derivatives (anti UV-A filters), wherein the function of the former is to prevent the photodegradation of the latter which, as known, are poorly light-stable (Int. J. Cosm. Science, 10, 53, 1988). Combinations of anti UV-B filters having triazine structure and dibenzoylmethane derivatives are also disclosed in EP 0 800 813 and EP 0 845 260.

The kind of sunscreens and the amounts at which they are used, alone or in mixtures, are selected depending on the intended protection, as well as on the above mentioned remarks. In particular, an index of this protection is the so-called sun protection factor, (SPF), which is expressed as the ratio of the time of irradiation necessary to reach the erythematogenic threshold in the presence of the anti UV filter to the time necessary to reach the erythematogenic threshold in the absence of the anti UV filter.

There is, anyway, a demand for novel, more effective protection systems for those parts of the body which can he damaged by a more or less prolonged exposition to UV radiations, such as skin and hair, which demand is even more felt nowadays in view of the problems deriving from the holes in the ozone layer.

Therefore, an object of the present invention are combinations of sunscreens comprising one or more anti UV-B sunscreens selected from compounds of formula I: 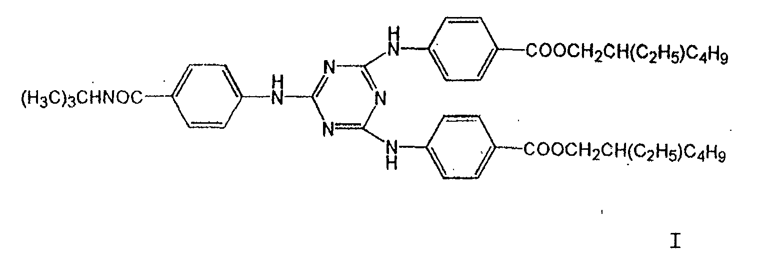 and/or the compound identified by CAS Registry Number 207174-74-1 and known under the trade name Parsol® SLX (hereinafter referred to as "compound II") and one or more anti UV-A sunscreens selected from the compounds of formula III and/or IV, V, VI:

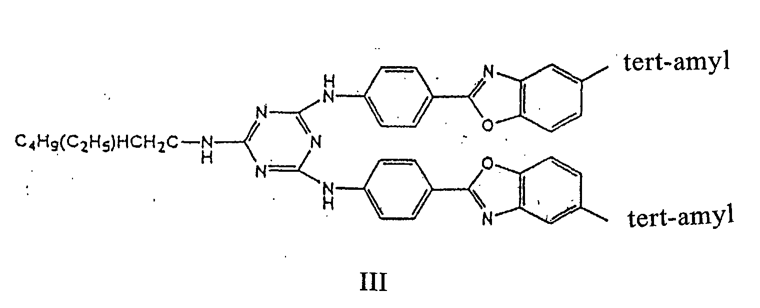

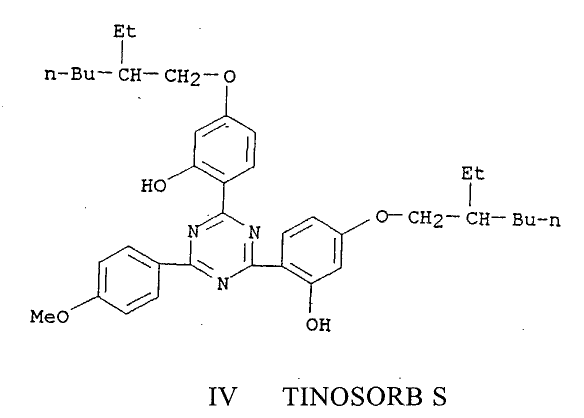

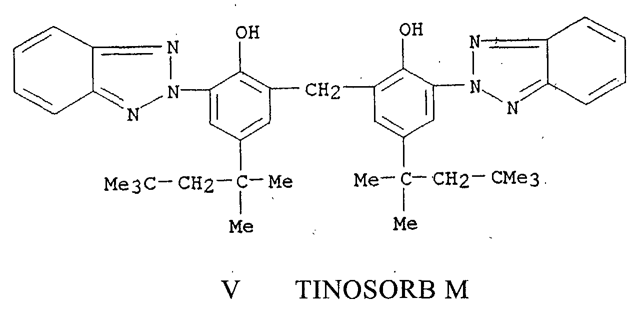

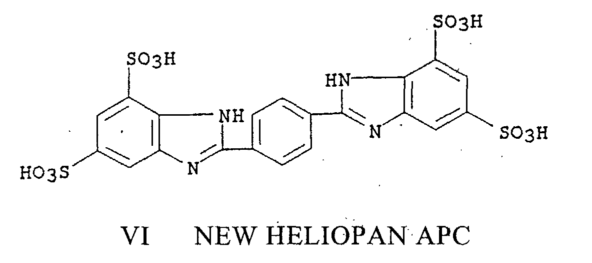

Preferred combinations according to the invention comprise at least one anti UV-B sunscreen selected from the compounds of formula I or II, and at least one anti UV-A sunscreen selected from the compounds of formula III, IV, V and VI. More preferred combinations are those which comprise, as anti UV-B sunscreen, the compound of formula I and at least one anti UV-A sunscreen selected from compounds of formula III, IV, V and VI.

In the combinations of the present invention, the ratio of one or more anti UV-A filters to one or more anti UV-B filters can range within wide limits. For example, it can range from about 0.05 to about 3 and, preferably, from about 0.1 to about 2.

These combinations of sunscreens substantially cover the entire range of UV radiations, and can be dissolved or suspended in oily or aqueous phases.

Therefore, the combinations of sunscreens of the present invention, dissolved or suspended in aqueous or oily phases, can be used for protecting the human body from the harmful effects of the ultraviolet sun radiation.

In this respect, it has surprisingly been found that in the combinations of the invention, the presence of the anti UV-A filter remarkably enhances the specific extinction in the UV-B region; it is therefore possible to prepare dermatological or cosmetic formulations with SPF definitely higher than that of corresponding compositions containing anti UV-B filters only, which means that said combinations exert a synergistic effect with regard to the SPF.

Preferred compositions according to the invention are cosmetic and dermatological compositions containing said combinations in amounts ranging from about 0.05 to about 30% on the composition total weight: these compositions are a further object of the present invention.

A further object of the present invention is the use of the combinations as defined above for the preparation of cosmetic and dermatological compositions able to protect the human skin from sun radiation.

In a preferred embodiment, the cosmetic compositions comprise a sunscreens combination according to the invention in cosmetically acceptable carriers, such as oil-in-water, water-in-oil, oil-water-oil, water-oil-water, water-in-silicon emulsions, oily solutions, lipidic fusions, aqueous, hydroalcoholic or anhydrous gels, alcoholic or water-alcoholic solutions, and the like. When the use of an oily substance is necessary for dissolving or dispersing one or more components of the selected combination, the oily substance is advantageously selected from:
i) hydrocarbons, such as paraffin, mineral oils and the like;
ii) natural oils, butters and waxes, such as avocado oil, sunflower oil, almond oil, apricot seed oil, karité butter, evening primrose oil, black currant oil, borage oil, jojoba oil, safflower oil, wheat germ oil, macadamia oil, rice bran oil, sesame oil, castor oil, coconut oil; olive, avocado and soy unsaponifiable matter; cocoa butter, beeswax, candelilla wax, carnauba wax and the like;
iii) silicon oils, such as dimethicones, cyclomethicones, dimethiconols, alkyldimethicones, and the like;
iv) esters of saturated or unsaturated, straight or branched C₁₋₂₅ aliphatic acids, or of aromatic or alkylaromatic acids, said acids being optionally hydroxylated and/or ethoxylated with mono- or polyhydroxylated, saturated or unsaturated, straight or branched C₁₋₂₅ aliphatic alcohols, such as, octyldodecyl-neopentanoate, pentaerythritol-dioleate, trimethylolpropantrioleate, triisostearyl-citrate, diacetin, triacetin, 2-ethyl-hexyl-acetate, neopentylglycol-oleate, triethyleneglycol-diacetate, isopropyl-myristate, isopropyl-palmitate, bis-diglyceryl-caprylate/caprate/isostearate/stearate/hydroxystearate, bis-diglyceryl adipate, dioctyl-maleate, di-(2-ethyl-hexyl)-malate, (C₁₂₋₁₅)alkyl-benzoates, cetylstearyl-octanoate, cetylstearylisononanoate, 2-ethyl-hexyl-palmitate, 2-ethyl-hexyl-stearate, C₈₋₁₀ triglycerides, PEG7-glycerylcocoate and the like;
v) amides, such as those mentioned in the published European patent application 0 748 623, in particular N,N-diethyl-methylbenzamides and ethyl 1-[(N-acetyl-N-butyl)amino]-propionate;
vi) alcohols containing from 6 to 35 carbon atoms, such as cetyl alcohol, stearyl alcohol, behenyl alcohol, octyldodecyl alcohol, 3,5,5-trimethyl-hexyl alcohol, 2-butoxyethanol, 2-phenoxyethanol, 2-ethyl-1,3-hexandiol and the like;
vii) ethers of fatty alcohols containing from 8 to 40 carbon atoms, such as di-n-octylether;
viii) glycol butylethers, such as propylene glycol tert-butylether, diethylenglycol butylether, (polypropyleneglycol)3-53 butyl ether and the like;
ix) esters of (C₁₋₆)alkylethers, such as diethylene glycol butyl ether acetate, propylene glycol methyl ether acetate and the like.

For the purposes of the present invention, the substances from i) to ix), easily available on the market, can be used singularly, or as admixture thereof, such as the mixtures of waxes commercialised under the name CUTINA™ (Henkel).

Generally, the oily component is used in amounts ranging from about 0.5 to 95% or more on the composition total weight.

A preferred group of oily components comprises esters of saturated or unsaturated, straight or branched C₁₋₂₅ aliphatic acids, or of aromatic or alkylaromatic acids, said acids being optionally hydroxylated and/or ethoxylated, with mono- or polyhydroxylated, saturated or unsaturated C₁₋₂₅ aliphatic alcohols.

A further preferred group of oily components comprises N,N-diethylmethylbenzamides and ethyl 1-(N-acetyl-N-butyl)-propionate.

A third preferred group of oily components comprises mixtures of esters of saturated or unsaturated, straight or branched C₁₋₂₅ aliphatic acids, or of aromatic or alkylaromatic acids, said acids being optionally hydroxylated and/or ethoxylated, with mono- o polyhydroxylated, saturated or unsaturated, straight or branched C₁₋₂₅ aliphatic alcohols, and N,N-diethylmethylbenzamides and/or ethyl 1-(N-acetyl-N-butyl)-propionate.

A fourth preferred group of oily components comprises silicon oils.

Cosmetic compositions comprising the above mentioned emulsions can also comprise one or more conventional emulsifiers available on the market. These can be anionic emulsifiers, such as fatty acids soaps with alkali (for example, potassium stearate), or alkaline-earth metals, or aliphatic amines; alkylsulfate salts, such as sodium cetylstearyl sulphate (LANETTE™ E, Henkel); ethoxylated and non- ethoxylated alkylphosphate salts, such as potassium cetylphosphate (AMPHISOL™ K, Givaudan); fatty acids condensed with hydrolysed proteins (LAMECREME™ LPM, Henkel); monoand diglycerides anionic esters (GRINDATEK™ CA-P, Grindsted Products Ltd) and the like. Amphoteric emulsifiers, for example phospholipids, such as lecithin, or non ionic emulsifiers can also be used. The latter are, for example, ethoxylated compounds of natural oil derivatives, such as hydrogenated (7)OE castor oil (ARLACEL™ 989, ICI); mono- and diglycerides of ethoxylated and non-ethoxylated fatty acids, such as glyceryl stearate (CUTINA™ GMS, Henkel) and glyceryl (20)OE stearate (CUTINA™ E-24, Henkel); ethoxylated (TWEEN™, ICI and CRILLET™, Croda) and non-ethoxylated (SPAN™, ICI and CRILL™, Croda) sorbitan esters; polyglycerol esters of fatty acids, such as triglyceryl diisostearate (LAMEFORM™ TGI, Henkel) and triglyceryl distearate (CITHROL™ 2623, Croda); glucose, methylglucose and saccharose esters with ethoxylated and non- ethoxylated fatty acids, such as methylglucose dioleate (GLUCATE™ DO, Amerchol) and methylglucose(20)OE sesquistearate (GLUCAMATE™ SS E-20, Amerchol); glucose ethers and oligomers thereof, optionally esterified with C₁₀₋₃₀ aliphatic acids, such as triglicerylmethylglucosedistearate (TegoCare™ 450, Goldschmidt), or etherified with C₈₋₃₀ aliphatic alcohols, such as cetylstearyl glucoside (MONTANOV™ 68, Seppic); ethoxylated fatty acids (MYRJ™, ICI); ethoxylated fatty alcohols (BRIJ™, ICI); lanolin and ethoxylated and non ethoxylated derivatives thereof, such as lanolin (30)OE (AQUALOSE™ L 30, Westbrook); alkylglycols/polyethyleneglycols copolymers, such as PEG-45/dodecylglycol copolymer (ELFACOS™ ST 9, Akzo); silicon emulsifiers (Silicon Fluid 3225 C, Dow Corning; ABIL™ WS05, Th. Goldschmidt AG), fluorinated emulsifiers (FOMBLIN™ Ausimont); hydroxystearic acid polymers esterified or etherified with polyoxyethyleneglycols (PEG) (ARLACEL™ p 135, ICI), polypropylene glycols (PPG), or PEG/PPG copolymers and the like. Instead of these conventional emulsifiers, use can also be advantageously made of cross-linked copolymers selected from
a) cross-linked copolymers of i) acrylic or methacrylic acids, ii) vinyl esters of highly branched trialkylacetic acids of formula VII 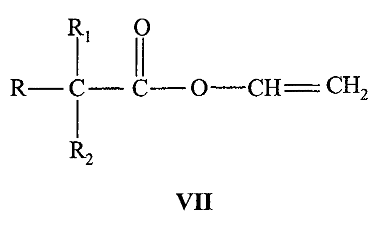 wherein R, R₁ and R₂ are straight alkyl residues, at least one of them being methyl, the total sum of the carbon atoms of the acyl residue being preferably 10, and iii) a cross-linking agent, such as pentaerythritol triallyl ether (Stabylen 30, 3V SIGMA-Bergamo, Italy), and
b) cross-linked copolymers of i) acrylic or methacrylic acids, ii) acrylates or methacrylates of C₁₀₋₃₀ aliphatic alkanols, and iii) a cross-linking agent, such as saccharose or pentaerythritol allyl ethers (PEMULEN™, B.F Goodrich).

The amount of emulsifiers, which can be used singularly or in combination, ranges from about 0.1 to about 20% on the composition total weight.

The cosmetic compositions of the invention can also comprise one or more vitamins, or precursors and analogues thereof, which can be advantageously selected from:
i) group A vitamins, vitamin A2 and retinal included, and esters thereof with straight or branched, saturated or unsaturated aliphatic C₂₋₂₀ monocarboxylic acids or C₃₋₁₂ di- or tricarboxylic acids, said acids being optionally hydroxylated or alkoxylated with PEG or PPG or PEG/PPG copolymers; or esters thereof with nicotinic acid;
ii) α- and β-caroten;
iii) group B vitamins;
iv) vitamin C and esters thereof with straight or branched, saturated or unsaturated aliphatic C₂₋₂₀ monocarboxylic acids or C₃₋₁₂ di- or tricarboxylic acids, said acids being optionally hydroxylated or alkoxylated with PEG or PPG or PEG/PPG copolymers; or esters thereof with nicotinic acid;
v) natural and synthetic tocopherols, vitamin E included, as racemates, or as optically active isomers, and esters thereof with straight or branched, saturated or unsaturated aliphatic C₂₋₂₀ monocarboxylic acids or C₃₋₁₂ di- or tricarboxylic acids, said acids being optionally hydroxylated or alkoxylated with PEG or PPG or PEG/PPG copolymers; or esters thereof with nicotinic acid;
vi) Essential Fatty Acids (EFA);
vii) vitamin H;
viii) P vitaminic complex;
ix ) vitamin PP.

Generally, the amount of vitamins, precursors or analogues thereof ranges within quite broad limits. Preferably, the amount ranges from about 0.02 to about 10% by weight, calculated on the composition total weight.

The cosmetic compositions comprising one or more of the above mentioned vitamins, or precursors or analogues thereof, are a further object of the present invention.

Finally, the combinations of sunscreens and the cosmetic compositions of the present invention can also comprise, in combination, one or more anti UV-A or anti UV-B sunscreens selected, for example, from benzylidenecamphor derivatives, dibenzoylmethane derivatives, alkoxycinnamic acids esters and salts, benzophenone derivatives, diphenylcyanoacrylates, salicylic acid derivatives, benzimidazolesulfonic acid derivatives, p-aminobenzoic acid derivatives, 2-(2H-benzotriazol-2-yl)-4-methyl-6-{2-methyl-3-[1,3,3,3-tetramethyl-1-[(trimethylsilyl)oxy]-disiloxanyl]-propyl}-phenol (silatriazole), and metal oxides having atomic number ranging from 21 to 30.

Representative examples of the sunscreens belonging to the above mentioned classes of compounds comprise benzylidenecamphor derivatives, such as bicyclo[2.2.1]heptan-2-one; 1,7,7-trimethyl-3-[(4-methylphenyl)-methylene]; 3-(4'-trimethylammonium)benzylidenebornan-2-one methylsulfate; and 3,3'-(1,4-phenylenedimethin)-bis-(7,7-dimethyl-2-oxobicyclo[2,2,1]heptan-1-methanesulfonic) acid, respectively commercially known as EUSOLEX™ 6300, MEXORIL™ SK and MEXORIL™ SX; 4-methoxy-4'-tert-butyl-dibenzoylmethane, the dibenzoylmethane derivative commercially known as PARSOL™ 1789; 2-ethylhexyl-4-methoxycinnamate and 4-metoxycinnamic acid diethanolamine salt, alkoxycinnamic acids derivatives commercially known as PARSOL™ MCX and BERNEL™ HYDRO; 2,4-dihydroxybenzophenone, 2-hydroxy-4-methoxy-benzophenone and 5-benzoyl-4-hydroxy-2-methoxy-benzenesulfonic acid, benzophenone derivatives commercially known as UVASORB™ 20H, UVASORB™ MET and UVASORB™ S5; 2-ethylhexyl 2-cyano-3,3-diphenylacrylate, commercially known as UVINUL™ N-539; 2-ethylhexyl 2-hydroxybenzoate, (+)-3,3,5-trimethylcyclohexyl salicylate and salicylic acid triethanolamine salt, salicylic acid derivatives commercially known as ESCALOL™ 587, KEMESTER™ HMS and SUNAROME™ W; 2-phenyl-5-benzimidazolylsulfonic acid, commercially known as EUSOLEX™ 232; p-aminobenzoic acid; 2-ethylhexyl 4-dimethylamino-benzoate, ethyl N,N-bis-(2-hydroxypropyl)-benzoate and 4-aminobenzoic acid PEG 25, p-aminobenzoic acid derivatives commercially known as UVASORB™ DMO, AMERSCREEN™ P and UVINUL™ P25.

Among metal oxides having atomic number ranging from 21 to 30, titanium dioxide (TiO₂) and zinc oxide (ZnO) are preferred. These oxides are preferably used in micronized form, in which the particles have a size not higher than about 100 nm as for TiO₂ and ranging from about 15 to about 300 nm as for ZnO. More preferably, TiO₂ particle size ranges from about 5 to about 50 nm. Titanium dioxide can have an anatase, rutile, or amorphous structure. These micronized metal oxides can be used as they are or coated with other agents, such as Al₂O₃ or aluminium salts with C₁₀₋₁₈ aliphatic fatty acids or silicones, commonly available on the market. For example, micronized TiO₂ is commercialised under the trade name P25 (Degussa), whereas TiO₂ coated with aluminium stearate is commercialised as MT100T (Taika Corp.), and TiO₂ coated with Al₂O₃ is known as UFTR (Miyoshi). Micronized ZnO is, in turn, marketed as Z-COTE™ (sunSMART) or as SPECTRAVEIL™ (Tioxide).

The cosmetic compositions of the invention can also comprise other conventional components, for example, emulsions stabilizing agents, such as sodium chloride or citrate, magnesium sulphate and analogues, in amounts ranging from about 0.1 to about 5% on the composition weight; wetting agents, such as glycerine, propylene glycol or 1,3-butyleneglycol, in amounts from about 0.1 to about 30% on the composition weight; thickening agents, such as modified cellulose, or acrylic acids polymers or copolymers, in amounts not higher than 4% on the composition weight; sequestering agents, such as EDTA salts, in amounts not higher than 1% on the composition weight; antioxidants, such as tocopherols and esters thereof, hydroxytoluene butoxide or butyl hydroxy anisole, in amounts not higher than 2% on the composition weight; emollients, such as mineral oils, polysiloxanes, almond oil, vaseline, isopropyl myristate or fatty acids triglycerids, in amounts comprised between about 0.1 and about 95% on the composition weight; moisturizing agents in amounts not higher than 5% on the composition weight; agents for adjusting pH to desired values, such as sodium or potassium citrate, sodium or potassium hydroxide, or citric acid monohydrate, in amounts not higher than 1% on the composition weight; filming agents, such as alkylated polyvinylpyrrolidones or high molecular weight waxes, in amounts not higher than 5% on the composition weight; preservatives, such as 2-bromo-2-nitro-propanediol, sodium dehydroacetate, isothiazolone, imidazolidinylurea, diazolidinylurea, parabens and idantoin derivatives (GLYDANT™ Lonza), sorbic acid, benzoic acid and salts thereof, chlorhexidine and salts thereof, phenoxyethanol, benzyl alcohol, and the like, in amounts not higher than 10% on the composition weight.

Perfumes and dyes can be added according to the state of the art. Moreover, the compositions of the invention can also contain an artificial tanning agent, such as dihydroxy acetone (DHA), optionally in the presence of an iron salt, as described in the published European patent application 0 688 203, in order to provide a less yellowish suntan shade than that provided by DHA only. These artificial tanning agents can be present in amounts ranging from about 0.1 to about 10% on the composition weight.

The cosmetic compositions of the invention can be prepared according to the procedures known to the expert in the art. For example, in the case of compositions in which the cosmetically acceptable carrier consists of an oil-in-water or water-in-oil emulsion, it is preferable to prepare the two phases separately, dissolving or dispersing in each phase the desired lipophilic or hydrophilic components/ingredients, and mix them afterwards.

Examples of said compositions are sun-creams, day creams, moisturizing creams, sun-oils, ointments, lipsticks, solutions, lotions, gels, transparent gels, aerosol, foams and the like.

The compositions contain a selected combination according to the present invention together with the other ingredients in the above mentioned weight ratios, as well as any other compatible ingredient conventionally used in cosmetic preparations. In general, said compositions comprise a selected combination of the present invention that provides a SPF not lower than 2. In several cases, the sunscreen combinations of the present invention have shown a synergistic effect.

Some representative examples of cosmetic compositions containing the combinations of invention are provided hereinbelow. The amount of the single components is expressed as weight percentages on the composition total weight.

### Example A

A facial day-cream is prepared with the following ingredients

| | | |
|---|---|---|
| 1. | Cetylstearyl glucoside | 4.00 |
| 2. | Glyceryl stearate | 1.00 |
| 3. | Cetylstearyl alcohol | 1.00 |
| 4 | C₁₂₋₁₅ alkyl benzoate | 5.00 |
| 5. | Octyl octanoate | 5.00 |
| 6. | Dimethicone | 0.50 |
| 7. | Compound of formula I | 1.00 |
| 8. | Compound of formula IV | 1.00 |
| 9. | Demineralised water | q.s. to 100 |
| 10. | Imidazolidinyl urea | 0.30 |
| 11. | Methyl p-hydroxy-benzoate | 0.20 |
| 12. | Glycerine | 3.00 |
| 13. | Perfume | 0.30 |

Ingredients 1 - 6 are mixed, melted and heated to 70°C and ingredients 7 and 8 are added thereto under vigorous stirring (phase A). Water is heated separately to 70°C (phase B). Phase A is then added to phase B with a turboemulsifier. After cooling the resulting emulsion to room temperature, glycerine, preservatives and perfume are added.

### Example B

An oil-in-water fluid emulsion is prepared with the following ingredients:

| | | |
|---|---|---|
| 1. | Sorbitan(20)OE stearate | 0.25 |
| 2. | Avocado oil | 3.00 |
| 3. | Octyl palmitate | 3.00 |
| 4. | Mineral oil | 3.00 |
| 5. | Compound of formula I | 3.00 |
| 6. | Compound of formula VI | 2.00 |
| 7. | Demineralised water | q.s. to 100 |
| 8. | Stabylen 30 (emulsifier polymer 3V SIGMA, Bergamo, Italy) | 0.25 |
| 9. | Sodium hydroxide | q.s. to pH 7 |
| 10. | Diazolidinyl urea | 0.30 |
| 11. | Isothiazolone | 0.05 |
| 12. | Propylene glycol | 3.00 |

Components 1 - 4 are mixed and heated to 60°C; components 5 and 6 are added thereto under vigorous stirring (phase A). Component 8 is dispersed separately under stirring in water at 60°C, (phase B). Phase A is then added to phase B with a turboemulsifier and pH is adjusted to 7 with sodium hydroxide. After cooling to room temperature, propylene glycol, preservatives and perfume are added.

### Example C

An anhydrous ointment is prepared with the following ingredients:

| | | |
|---|---|---|
| 1. | Mineral oil | q.s. to 100 |
| 2. | Cetyl stearyl isononanoate | 30.00 |
| 3 | C₈₋₁₀ triglyceride | 30.00 |
| 4. | Compound of formula II | 5.00 |
| 5. | Compound of formula III | 1.00 |
| 6. | Micronized titanium dioxide | 2.00 |
| 7. | Hydrogenated castor oil | 1.50 |
| 8. | Pyrogenic silica | 1.50 |
| 9. | Perfume | 0.30 |

Ingredients 1, 2 and 3 are mixed, heated to 60°C and components 4 and 5 are added thereto under stirring. Ingredients 6, 7 and 8 are then dispersed in the mixture under vigorous stirring. The mixture is finally cooled to room temperature and added with perfume.

### Example D

A water-in-oil fluid emulsion is prepared with the following ingredients:

| | | |
|---|---|---|
| 1. | Hydrogenated castor oil (7)OE | 7.50 |
| 2. | Lanolin alcohols in mineral oil | 2.50 |
| 3. | Hydrogenated polyisobutene | 5.00 |
| 4. | Octyl octanoate | 5.00 |
| 5. | C₈₋₁₀ triglyceride | 3.00 |
| 6. | Compound of formula I | 3.00 |
| 7. | Compound of formula III | 2.00 |
| 8. | Compound of formula V | 2.00 |
| 9. | 4-Methoxy-4'-tert-butyldibenzoylmethane (PARSOL™ 1789) | 2.00 |
| 10. | Demineralised water | q.s. to 100 |
| 11. | Dimethyl-dimethylol-idantoin | 0.30 |
| 12. | Phenoxyethanol and parabens | 1.00 |
| 13. | Glycerine | 5.00 |
| 14. | Perfume | 0.30 |

Ingredients 1 - 5 are mixed and heated to 70°C; ingredients 6, 7, 8 and 9 are added thereto under stirring (phase A). Water is heated separately to 70°C and added to phase A with a turboemulsifier. The mixture is then cooled to room temperature, added with the preservatives previously mixed to glycerine and with perfume.

### Example E

A high protection water-in-oil cream is prepared is prepared with the following ingredients:

| | | |
|---|---|---|
| 1. | Triglyceryl diisostearate | 4.00 |
| 2. | Beeswax | 2.00 |
| 3. | Mineral oil | 10.00 |
| 4. | Octyl octanoate | 5.00 |
| 5. | Cyclomethicone | 5.00 |
| 6. | Compound of formula I | 3.00 |
| 7. | Compound of formula II | 2.00 |
| 8. | Compound of formula VI | 5.00 |
| 9. | Hydrogenated castor oil | 0.50 |
| 10. | Demineralised water | q.s. to 100 |
| 11. | Sodium dehydroacetate | 0.30 |
| 12. | Phenoxyethanol and parabens | 1.00 |
| 13. | Glycerine | 5.00 |
| 14. | Perfume | 0.30 |

Ingredients 1 - 5 are mixed, melted at 60°C and added with ingredients 6 - 8 (phase A), under stirring. Ingredient 9 is then dispersed therein with a turboemulsifier. Water is heated separately to 60°C and added to phase A with a turboemulsifier. The cream is cooled to room temperature and added with preservatives, glycerine and perfume.

### Example F

An oil-in-water cream is prepared with the following ingredients:

| | | |
|---|---|---|
| 1. | Cetyl stearyl alcohol (33)OE | 2.50 |
| 2. | Glyceryl stearate (20)OE | 2.00 |
| 3. | Behenyl alcohol | 2.00 |
| 4. | Isohexadecane | 10.00 |
| 5. | Safflower oil | 3.00 |
| 6. | Borage oil | 2.00 |
| 7. | Butyl hydroxyanisole | 0.10 |
| 8. | PVP/eicosene copolymer | 1.00 |
| 9. | Compound of formula I | 3.00 |
| 10. | Compound of formula V | 1.00 |
| 11. | Compound of formula VI | 1.00 |
| 12. | EUSOLEX™ 6300 | 1.00 |
| 13. | Micronized zinc oxide | 2.00 |
| 14. | Demineralised water | q.s. to 100 |
| 15. | SYNTHALEN™ K (Carbomer 940 - 3V SIGMA, Bergamo, Italy) | 0.10 |
| 16. | Amino methyl propanol | 0.10 |
| 17. | Imidazolidinyl urea | 0.30 |
| 18. | Phenoxyethanol and parabens | 1.00 |
| 19. | Butyleneglycol | 2.00 |
| 20. | Perfume | 0.30 |

Ingredients 1 - 8 are mixed and melted to 70°C and ingredients 9 - 13 are added thereto under vigorous stirring (phase A). Ingredient 15 is separately dispersed under stirring in water at 70°C (phase B). Phase A is then added to phase B with a turboemulsifier, and the resulting mixture is then neutralized to pH 7 with ingredient 16. After cooling to room temperature preservatives, butyleneglycol and perfume are added.

### Example G

A sun oil is prepared with the following ingredients:

| | | |
|---|---|---|
| 1. | Dioctyl cyclohexane | q.s. to 100 |
| 2. | C₈₋₁₀ triglyceride | 20.00 |
| 3. | Isopropyl palmitate | 30.00 |
| 4. | Cyclomethicone | 10.00 |
| 5. | Perfume | 0.30 |
| 6. | Compound of formula I | 3.00 |
| 7. | Compound of formula IV | 2.00 |

Ingredients 6 and 7 are added under stirring to a mixture of ingredients 1, 2 and 3. The resulting mixture is heated to dissolution of ingredients 6 and 7. After cooling to room temperature, ingredients 4 and 5 are added.

## Claims

1. Combinations of sunscreens comprising one or more anti UV-B sunscreens selected from compound of formula I 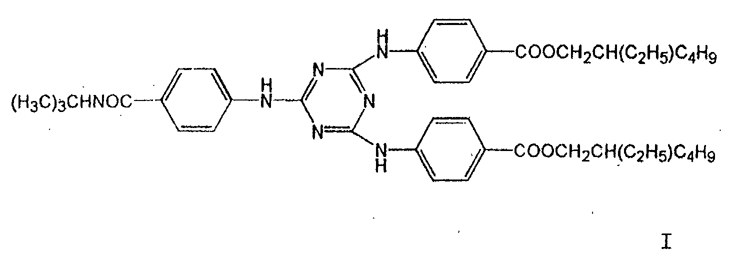 and compound of formula II, identified with CAS Registry Number 207174-74-1 and known under the trade name Parsol® SLX,
and one or more anti UV-A sunscreens selected from the compounds of formula III and/or IV, V, VI:

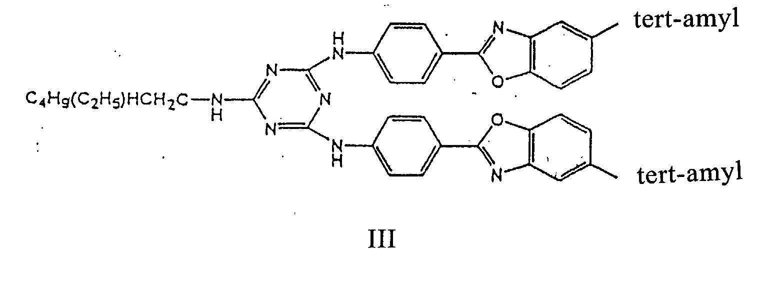

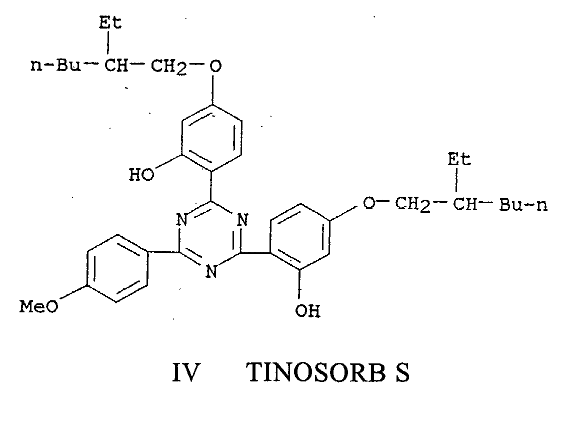

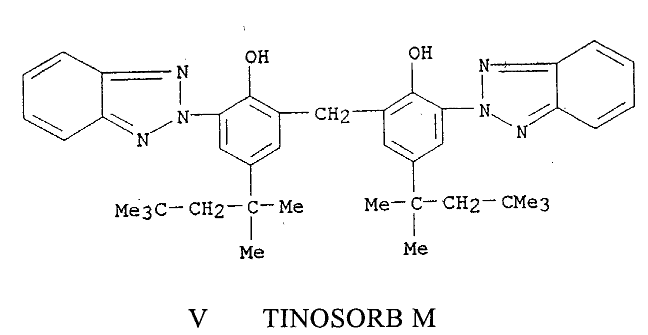

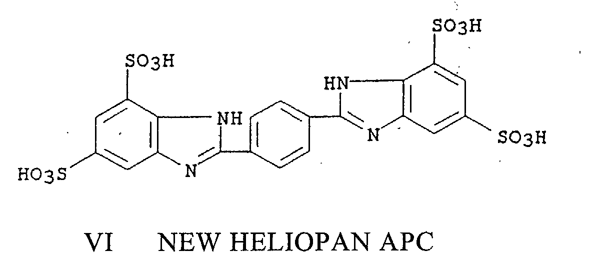

2. Compositions according to claim 1, wherein the ratio of the total amount of anti UV-A filters to the total amount of anti UV-B filters ranges from 0.05 to 3.

3. Compositions according to claim 2, wherein the ratio of one or more of the anti UV-A filters to one or more anti UV-B filters ranges from 0.1 to 2.

4. Cosmetic and dermatological compositions containing a combination according to any one of claims 1 - 3 in amounts ranging from 0.05 to 30% on the composition total weight.

5. Cosmetic compositions according to claim 4, **characterised in that** they are oil-in-water, water-in-oil, oil-water-oil, water-oil-water, water-in-silicon emulsions, oily solutions, lipidic fusions; aqueous, hydroalcoholic or anhydrous gels; alcoholic or water-alcoholic solutions.

6. Cosmetic compositions according to any one of claims 4 to 5, wherein the solvent or the dispersing agent of the oily phase is selected from hydrocarbons; oils, butters and natural waxes; silicon oils; esters of saturated or unsaturated, straight or branched C₁₋₂₅ aliphatic acids, or of aromatic or alkylaromatic acids, said acids being optionally hydroxylated and/or ethoxylated with mono- or polyhydroxylated, saturated or unsaturated, straight or branched C₁₋₂₅ aliphatic alcohols; amides; C₆₋₃₅ alcohols; ethers of C₈₋₄₀ fatty alcohols; glycol butyl ethers or (C₁₋₆)alkylethers esters.

7. Cosmetic compositions according to any one of claims 4 - 6, wherein the solvent or dispersing agent of the oily phase is selected from esters of saturated or unsaturated, straight or branched C₁₋₂₅ aliphatic acids or of aromatic or alkylaromatic acids, said acids being optionally hydroxylated and/or ethoxylated, with mono- or polyhydroxylated, saturated or unsaturated, straight or branched C₁₋₂₅ aliphatic alcohols, N,N-diethyl-methylbenzamides or ethyl 1-(N-acetyl-N-butyl)-propionate, or mixtures thereof.

8. Cosmetic compositions according to any one of claims 4 - 6, wherein the solvent or the dispersing agent of the oily phase is a silicone oil.

9. Cosmetic compositions according to any one of claims 4 - 8, wherein the solvent or the dispersing agent of the oily phase is used in amounts ranging from 0.5 to 95% on the composition total weight.

10. Compositions according to claims 4 - 9, comprising vitamins, precursors and analogues thereof selected from:
i) group A vitamins, vitamin A₂ and retinal included, and esters thereof with straight or branched, saturated or unsaturated aliphatic C₂₋₂₀ monocarboxylic acids or C₃₋₁₂ di- or tricarboxylic acids, said acids being optionally hydroxylated or alkoxylated with PEG, PPG or PEG/PPG copolymers; or esters thereof with nicotinic acid;
ii) α- and β-caroten;
iii) group B vitamins;
iv) vitamin C, and esters thereof with straight or branched, saturated or unsaturated aliphatic C₂₋₂₀ monocarboxylic acids or C₃₋₁₂ di- or tricarboxylic acids, said acids being optionally hydroxylated or alkoxylated with PEG or PPG or PEG/PPG copolymers or esters thereof with nicotinic acid;
v) natural and synthetic tocopherols, as racemates or as optically active isomers, and esters thereof with straight or branched, saturated or unsaturated aliphatic C₂₋₂₀ monocarboxylic acids or C₃₋₁₂ di- or tri-carboxylic aliphatic acids, said acids being optionally hydroxylated or alkoxylated with PEG, PPG or PEG/PPG copolymers; or esters thereof with nicotinic acid;
vi) Essential Fatty Acids;
vii) vitamin H;
viii) P vitaminic complex; and
ix) vitamin PP.

11. Compositions as claimed in any one of claims 4 - 10, wherein the vitamins, precursors and analogues thereof are used in amounts ranging from 0.02 to 10% by weight on the composition total weight.

12. Compositions according to any one of claims 4 - 9, comprising one or more anti UV-A or anti UV-B sunscreens selected from benzylidenecamphor derivatives, dibenzoylmethane derivatives, alkoxycinnamic esters and salts, benzophenone derivatives, diphenylcyanoacrylates, salicylic acid derivatives, benzymidazolesulfonic acid derivatives, p-aminobenzoic acid derivatives, 2-(2H-benzotriazol-2-yl)-4-methyl-6-{2-methyl-3-[1,3,3,3-tetramethyl-1-[(trimethylsilyl)oxy]disiloxanyl]propyl}phenol, and metal oxides having atomic number ranging from 21 to 30.

13. Use of the combinations according to any one of claims 1 - 3 for the preparation of cosmetic or dermatological compositions able to protect the human skin from sun radiation.
